# EUROPÄISCHE PATENTSCHRIFT

(11) **EP 1 028 725 B1**
(45) Veröffentlichungstag und Bekanntmachung des Hinweises auf die Patenterteilung: **11.02.2004**
(21) Anmeldenummer: 98962320.2
(22) Anmeldetag: 04.11.1998
(51) Int. Cl.: A61K 31/404, A61K 31/55, A61K 38/08, A61P 27/00, A61P 43/00, A61K 45/00, A61K 45/06

(54) **VERWENDUNG VON VASOPRESSIN-ANTAGONISTEN ZUR BEHANDLUNG VON STÖRUNGEN ODER ERKRANKUNGEN DES INNENOHRES**
USE OF VASOPRESSIN ANTAGONISTS FOR TREATING DISTURBANCES OR ILLNESSES OF THE INNER EAR
UTILISATION D'ANTAGONISTES DE LA VASOPRESSINE POUR LE TRAITEMENT DE TROUBLES OU DE MALADIES DE L'OREILLE INTERNE

(30) Priorität: 05.11.1997 DE 19748763
(43) Veröffentlichungstag der Anmeldung: 23.08.2000
(73) Patentinhaber: Otogene Aktiengesellschaft, 72076 Tübingen (DE)
(72) Erfinder: ZENNER, Hans, Peter, D-72070 Tübingen (DE); RUPPERSBERG, J., Peter, D-72072 Tübingen (DE); LÖWENHEIM, Hubert, D-72076 Tübingen (DE)
(74) Vertreter: Patentanwälte Ruff, Wilhelm, Beier, Dauster & Partner
(86) Internationale Anmeldenummer: PCT/EP1998/007033
(87) Internationale Veröffentlichungsnummer: WO 1999/024051

(56) Entgegenhaltungen:
- MANTIONE C R ET AL: "A bradykinin (BK)1 receptor antagonist blocks capsaicin-induced ear inflammation in mice." BRITISH JOURNAL OF PHARMACOLOGY, (1990 MAR) 99 (3) 516-8, XP002101761
- CHEMICAL ABSTRACTS, vol. 108, no. 1, 4. Januar 1988 Columbus, Ohio, US; abstract no. 887, MORI, NOZOMU ET AL: "The study on vasopressin receptors in the cochlea" XP002101764 & EAR RES. JPN. (1987), 18, 44-6,
- YAMAMURA Y ET AL: "Characterization of a novel aquaretic agent, OPC- 31260, as an orally effective, nonpeptide vasopressin V2 receptor antagonist." BRITISH JOURNAL OF PHARMACOLOGY, (1992 APR) 105 (4) 787-91, XP002101762 in der Anmeldung erwähnt
- SERRADEIL-LE GAL C ET AL: "Characterization of SR 121463A, a highly potent and selective, orally active vasopressin V2 receptor antagonist." JOURNAL OF CLINICAL INVESTIGATION, (1996 DEC 15) 98 (12) 2729-38, XP002101763 in der Anmeldung erwähnt
- DATABASE MEDLINE US NATIONAL LIBRARY OF MEDICINE (NLM), BETHESDA, MD, US MANNING M ET AL: "Novel linear antagonists of the antidiuretic (V2) and vasopressor (V1) responses to vasopressin." XP002101765 in der Anmeldung erwähnt & INTERNATIONAL JOURNAL OF PEPTIDE AND PROTEIN RESEARCH, (1988 DEC) 32 (6) 455-67,

## Beschreibung

Die Erfindung betrifft die Verwendung mindestens eines Vasopressin-Rezeptorantagonisten oder deren Mischungen zur Herstellung eines Medikamentes zur Behandlung von Störungen oder Erkrankungen des Innenohres.

Vasopressin (VP) ist bekanntlich ein Peptidhormon aus dem Hypophysen-Hinterlappen. Aufgrund seiner antidiuretischen Wirkung wird es auch als Antidiuretin oder antidiuretisches Hormon (ADH) bezeichnet. Die beim Menschen und vielen Säugetieren vorkommende Form des Hormons ist ein cyclisches Peptid aus neun Aminosäuren mit einer Disulfid-Brücke, bei dem in 8-Stellung Arginin sitzt. Dementsprechend wird diese Form auch Arginin-Vasopressin (AVP) genannt.

Wie bereits erwähnt, ist der Einfluß von Vasopressin bei der Wasserdiurese in den Nieren, nämlich seine dabei entfaltete antidiuretische Wirkung, physiologisch besonders wichtig. Vasopressin macht die sog. Sammelrohre in der Niere wasserdurchlässig und ermöglicht auf diese Weise die Rückresorption von Wasser in den Nieren und damit das Aufkonzentrieren des Urins. Hierbei reagieren die Epithelien der Sammelrohre auf die Anwesenheit von Vasopressin. Das von der Blutseite der Epithelzellen herangeführte Hormon bindet dabei an spezifische Rezeptoren und stimuliert über intrazelluläres cAMP (second messenger cyclisches Adenosin-3',5-Monophosphat) die Zunahme der Wasserpermeabilität. Den zugrundeliegenden Mechanismus kann man sich so vorstellen, daß in den sog. Hauptzellen wasserkanalbildende Glykoproteine gebildet werden. Dieses Glykoprotein ist im Fall der Hauptzellen des Sammelrohrs der Niere das bisher ausschließlich dort nachgewiesene Aquaporin-2. Dieses wird zunächst in kleinen Vesikeln im Zellinneren gespeichert und bei Anwesenheit von Vasopressin am Rezeptor in die apikale Zellmembran eingebaut. Dadurch wird der hormonell regulierte Wassereintritt in die Zelle ermöglicht.

Vasopressin-Rezeptoren, die eine cAMP-abhängige Wasserkanalregulation in den Epithelzellen der Sammelrohre in der Niere vermitteln, werden als V₂-Rezeptoren bezeichnet.

Vasopressin besitzt also in den Epithelzellen der Sammelrohre der Niere eine Wasser rückresorbierende Wirkung. Diese kann durch Vasopressin-Rezeptorantagonisten gehemmt werden. Dementsprechend wirken diese Antagonisten in der Niere der Wirkung des Vasopressins entgegen und erhöhen somit den Urinfluß bei gleichzeitiger Verdünnung des Urins.

Im Zusammenhang mit der antidiuretischen Wirkung des Vasopressins sind bereits Vasopressin-Rezeptorantagonisten bekannt. Hierbei kann es sich um peptidische oder um nicht-peptidische Substanzen handeln. Bezüglich der peptidischen Substanzen wird auf die Veröffentlichungen von M. Manning und W.H. Sawyer in J. Lab. Clin. Med. 114, 617 - 632 (1989) und F.A. Laszlo et al. in Pharmacol. Rev., 43, 73 - 108 (1991) verwiesen. Darstellungen von nicht-peptidischen Substanzen finden sich bei Y. Yamamura et al. in Br. J. Pharmacol. 105, 787 - 791 (1992) und C. Serradeil-Le Gal et al. in J. Clin. Invest., 98 (12), 2729 - 2738 (1996). Alle diese Substanzen wurden in Bezug auf die antidiuretische Wirkung des Vasopressins untersucht und verwendet.

Bisherige Befunde und Untersuchungen über Störungen oder Erkrankungen des Innenohres lassen sich jedoch mit den oben beschriebenen Erkenntnissen über die antidiuretische Wirkung des Vasopressins und die Hemmung dieser Wirkung durch Antagonisten nicht in Einklang bringen. Dies betrifft insbesondere auch den sog. Endolymphhydrops im Innenohr, bei dem es zu einem Flüssigkeitsüberschuß der Endolymphe im Endolymphraum des Innenohres kommt. Dieser Endolymphhydrops kann mit einer Überproduktion oder einer Abflußstörung der Endolymphe insbesondere im sog. endolymphatischen Sack (Saccus endolymphaticus) im Zusammenhang stehen. Obwohl die Existenz von Vasopressin im Innenohr nachgewiesen ist, kann ein Einsatz von Vasopressin-Antagonisten aufgrund der bisherigen Erkenntnisse über die Wasser rückresorbierende Wirkung des Vasopressins nicht in Frage kommen. Bei einem erhöhten Flüssigkeitsvolumen im Innenohr, das die Krankheitssymptome auslösen kann, wäre die bekannte Wirkung des Vasopressins erwünscht. Durch den Einsatz des Antagonisten würde diese Wirkung gehemmt.

Es wurde nun überraschend gefunden, daß im Innenohr, insbesondere im Epithel von Zellen, die die Endolymphe einschließen, die Wasserdurchlässigkeit durch den Einsatz von Vasopressin-Rezeptorantagonisten hergestellt oder verbessert werden kann. Durch diese unerwartete, entgegengesetzte Wirkung des Antagonisten im Vergleich zu seiner Wirkung in der Niere wird die Verwendung solcher Substanzen oder deren Mischungen zur Behandlung von Störungen oder Erkrankungen des Innenohres ermöglicht.

Dementsprechend wird die erfindungsgemäße Aufgabe, Wirkstoffe zur Behandlung von Störungen oder Erkrankungen des Innenohres zugänglich zu machen, durch die Verwendung gemäß Anspruch 1 gelöst. Bevorzugte Ausführungen sind in den abhängigen Ansprüchen 2 bis 12 genannt. Der Inhalt aller dieser Ansprüche wird hiermit durch Bezugnahme zum Inhalt der Beschreibung gemacht.

Erfindungsgemäß kann mindestens ein Vasopressin-Rezeptorantagonist oder deren Mischungen zur Behandlung von Störungen oder Erkrankungen des Innenohres verwendet werden. Dabei ist die Verwendung zur Herstellung eines entsprechenden Medikamentes oder einer entsprechenden pharmazeutischen Zusammensetzung umfaßt, wobei der Antagonist ggf. auch in Form eines seiner pharmazeutisch akzeptablen Salze und ggf. in Mischung mit einem pharmazeutisch akzeptablen Träger oder Verdünnungsmittel eingesetzt werden kann.

Bei den erfindungsgemäß verwendeten Rezeptorantagonisten handelt es sich vorzugsweise um solche, die mit einem der oben genannten V₂-Rezeptoren wechselwirken. Diese V₂-Rezeptoren sind nach derzeitiger Kenntnis diejenigen, die in erster Linie mit der antidiuretischen Wirkung des Vasopressins im Zusammenhang stehen.

Die Störung oder Erkrankung des Innenohres, die bei der erfindungsgemäßen Verwendung behandelt werden soll, ist vorzugsweise zumindest mit einem der Symptome Schwindel (Gleichgewichtsstörungen), Schwerhörigkeit, Tinnitus (Ohrgeräusche) oder einem Druckgefühl im Ohr verbunden. Die Symptome Schwindel, Schwerhörigkeit oder Tinnitus sind hier besonders hervorzuheben. Bei der erfindungsgemäßen Verwendung kann eines der genannten Symptome allein auftreten, es ist jedoch eine beliebige Kombination von zwei oder drei Symptomen oder auch das Auftreten aller drei bzw. vier Symptome bei Innenohrstörungen typisch.

Das Symptom der Schwerhörigkeit kann insbesondere als sogenannte Tieftonschwerhörigkeit, und dabei vorzugsweise als fluktuierende Tieftonschwerhörigkeit auftreten.

Die durch die erfindungsgemäße Verwendung behandelbaren Störungen oder Erkrankungen des Innenohres lassen sich nach derzeitiger Kenntnis häufig und vorzugsweise mit einem sogenannten Hydrops, insbesondere einem Endolymphhydrops in Zusammenhang bringen. Bekanntlich handelt es sich bei einem Hydrops um eine Flüssigkeitsansammlung oder einen Flüssigkeitsstau im Körper, insbesondere in dort vorhandenen Hohlräumen. Bei dem oben bereits erwähnten Endolymphhydrops handelt es sich um einen Flüssigkeitsüberschuß der sogenannten Endolymphe. Dieser Flüssigkeitsüberschuß kann auf eine Überproduktion oder eine Abflußstörung der Endolymphe, insbesondere im sogenannten Saccus endolymphaticus, zurückzuführen sein. Der Endolymphhydrops resultiert in einem erhöhten Druck und einer Volumenzunahme des Raumes, in dem sich die Endolymphe befindet. Da damit eine veränderte Auslenkbarkeit der Sinneshärchen, die für das Hören und den Gleichgewichtssinn verantwortlich sind, im Zusammenhang steht, können die erwähnten Symptome, insbesondere Schwindel, Schwerhörigkeit und Tinnitus, mit einem Endolymphhydrops erklärt werden.

Von den behandelbaren Störungen bzw. Krankheiten sind insbesondere der sogenannte Morbus Ménière, der üblicherweise mit den Symptomen Schwindel, Schwerhörigkeit und Tinnitus (Ohrgeräusch) verbunden ist, zu nennen. Als Auslöser für Morbus Ménière können verschiedene Einflüsse in Frage kommen, wie beispielsweise auch Stress, Infektionen, Tumore, immunologische oder neurogene Störungen u.v.m.. Morbus Ménière ist hier als eine Art Sammelbezeichnung für Störungen zu verstehen, bei denen die entsprechenden Symptome in unterschiedlicher Ausprägung auftreten können, wie beispielsweise als vestibulärer Morbus Ménière. Auch die sogenannte Morbus Lermoyez ist als mögliche Anwendung zu nennen. Weiter können vorzugsweise Störungen/Krankheiten des Innenohrs behandelbar sein, die sich in einer Tieftonschwerhörigkeit äußern. Entsprechende Tieftonschwerhörigkeiten treten häufig auch auf nach entzündlichen Krankheiten, wie schleichender Mittelohrentzündung oder Syphilis, bei toxischen Einflüssen oder als "delayed-Hydrops-Syndrom" oder auch als Folge einer venösen Stauung (Stase) oder vaskulären Störungen des Innenohrs. Auch alle Störungen/Krankheiten des Innenohrs, die sich zusätzlich zu den bereits genannten mit Abflußstörungen der Endolymphe im Saccus endolymphaticus in Zusammenhang bringen lassen, sind ggf. besonders für einen Einsatz der vorliegenden Erfindung geeignet.

Erfindungsgemäß können bereits bekannte oder auch weitere neue Vasopressin-Rezeptorantagonisten, insbesondere Vasopressin-V₂-Rezeptorantagonisten, eingesetzt werden. Bei derartigen Substanzen kann es sich wie bei dem Vasopressin selbst um Peptidverbindungen handeln, die wie das Vasopressin mit dem Rezeptor wechselwirken. Derartige Peptidverbindungen sind beispielsweise in der bereits erwähnten Publikation von M. Manning und W.H. Sawyer offenbart. Dabei kann es sich insbesondere um vergleichsweise leicht zugängliche lineare Peptide handeln, wobei insbesondere das Peptid Propionyl-D-Tyr(Et)-Phe-Val-Asn-Abu-Pro-Arg-Arg-NH₂ eingesetzt wird. Die Bausteine der wiedergegebenen Peptidfolge besitzen dabei die in der Biochemie übliche Bedeutung, wobei es sich bei Abu um α-L-Aminobuttersäure handelt. Eine Auswahl grundsätzlich als Vasopressin-Rezeptorantagonisten einsetzbarer linearer Peptidverbindungen, einschließlich der besonders hervorgehobenen Verbindung, sind in der Veröffentlichung von M. Manning et al. in Int. J. Peptide Protein Res. 32, 455-467 (1988) genannt. Die mit ihrer Peptidfolge oben wiedergegebene Verbindung wird von der Fa. BACHEM Feinchemikalien AG, Bubendorf, Schweiz, unter der Produkt-Nr. H-9400 vertrieben.

Grundsätzlich ebenfalls einsetzbar sind nicht-peptidische Rezeptorantagonisten für Vasopressin, wobei es sich vorzugsweise um nicht-peptidische organische Substanzen handelt, die wiederum vorzugsweise synthetisch hergestellt sind. Bei den bisher bekannten organischen Substanzen kann es sich um Benzazepin-Derivate handeln, wie sie beispielsweise in der EP-A1-514667 beschrieben sind. Besonders hervorzuheben ist auch die in der Veröffentlichung von Y. Yamamura et al. in Br. J. Pharmacol., 105, 787-791 (1992) unter der Bezeichnung OPC-31260 beschriebene Substanz 5-Dimethylamino-1-{4-(2methylbenzoylamino)benzoyl}-2,3,4,5-tetrahydro-1H-benzazepin. Weiter sind als mögliche nicht-peptidische organische Substanzen Indol-Derivate geeignet, wie sie grundsätzlich aus der WO 93/15051, der WO 95/18105 und der EP-A1-645375 hervorgehen. Als N-Sulfonyl-2-Oxoindol-Derivat ist insbesondere das in J. Clin. Invest. 98 (12), 2729-2738 (1996) unter der Bezeichnung SR 121463A beschriebene 1-[4-(Ntert-butylcarbamoyl)-2-methoxybenzolsulfonyl]-5-ethoxy-3-spiro-[4-(2-morpholinoethoxy)cyclohexan]indol-2-one,fumarat zu nennen.

Weiter ist es erfindungsgemäß bevorzugt, daß der Rezeptorantagonist oral und/oder intravenös verabreichbar ist. Insbesondere eine orale Verabreichungsmöglichkeit, wie sie gerade nicht-peptidische Rezeptorantagonisten gegenüber den peptidischen Rezeptorantagonisten für Vasopressin aufweisen, ist besonders günstig, da hierdurch die Verabreichbarkeit an einen Patienten wesentlich erleichtert wird.

Die erfindungsgemäße Verwendung der Vasopressin-Rezeptorantagonisten kann grundsätzlich auf beliebige Weise erfolgen, wobei die gewählte Verabreichungsform auch dem Alter, dem Geschlecht oder anderen Charakteristika der Patienten, der Schwere der Störungen/Krankheiten und anderen Parametern angepaßt sein kann. Bei Verwendung zur oralen Verabreichung können beispielsweise grundsätzlich Tabletten, Pillen, Lösungen, Suspensionen, Emulsionen, Granulate oder Kapseln hergestellt werden. Hier können übliche pharmazeutische Träger, Verdünnungsmittel oder übliche Additive vorhanden sein. Zur intravenösen Verabreichung können die Antagonisten allein oder zusammen mit üblichen Hilfsflüssigkeiten, wie beispielsweise Glukose, Aminosäurelösungen und dgl. bereitgestellt werden. Auch eine Bereitstellung für die intramuskuläre, die subkutane oder intraperitoneale Verabreichung ist ggf. möglich. Auch an eine Verabreichung in Form von Suppositorien ist zu denken.

Die Dosierung ist ebenfalls in Abhängigkeit vom Krankheitsbild und von der Konditionierung des Patienten grundsätzlich frei wählbar. Üblicherweise können Mengen von 0,1 bis 50 mg/kg Körpergewicht und pro Tag vorgesehen sein. Pro Dosierungseinheit ist der Rezeptorantagonist für Vasopressin üblicherweise in einer Menge von etwa 10 bis 1.000 mg pro Einheit enthalten. In einer zur Verabreichung vorgesehenen Zubereitung bzw. einem entsprechenden Medikament ist der Rezeptorantagonist für das Vasopressin vorzugsweise in einer Menge von 1 bis 75 Gew.-% enthalten. Innerhalb dieses Bereiches sind Werte zwischen 5 und 50 Gew.-%, insbesondere 5 bis 25 Gew.-%, bevorzugt.

Die Anwendung einer erfindungsgemäß hergestellten Zubereitung bzw. eines entsprechenden Medikaments erfolgt grundsätzlich systemisch, wobei die bereits erwähnte orale Route bevorzugt ist. Unter Umständen kann auch eine lokale Anwendung in Richtung auf das Innenohr möglich sein, wenn beispielsweise durch eine Operation ein Zugang zum Innenohr hergestellt werden kann. So ist das Legen einer Drainage nach Exposition des Saccus endolympathicus möglich, wobei dann beispielsweise mit Hilfe einer Pumpe über einen entsprechenden Katheter der Vasopressin-Rezeptorantagonist direkt an den Wirkort einer entsprechenden Innenohrstörung/-erkrankung geführt wird.

In den Abbildungen zeigen:
- Abb. 1: die Lage der Reissner-Membran in der Cochlea in erwachsenen Meerschweinchen
a ohne Vasopressin-Zugabe
b bei chronischer Vasopressin-Zugabe
c bei akuter Vasopressin-Zugabe
d bei akuter Vasopressin-Zugabe (Ausschnitt-Vergrößerung)
- Abb. 2: Expression von
a V₂-Rezeptor und
b Aquaporin-2 im Epithel des endolymphatischen Sacks im Innenohr der Ratte.
- Abb. 3: Autoradiographie des menschlichen endolymphatischen Sacks
c im Epithel mit ¹²⁵I-Vasopressin
d Kontrollversuch in Anwesenheit von unmarkiertem Vasopressin.
- Abb. 4: organotypische Kultur des endolymphatischen Sacks der Ratte
a Übersichtsaufnahme
b Infrarotlicht-Mikroskopie
c SEM-Aufnahme
d SEM-Aufnahme (höhere Vergrößerung).
- Abb. 5: Membranumsatz in der Kultur gemäß Abb. 4
a FITC-Dextran-markierte Endosome in Abwesenheit von Vasopressin
b FITC-Dextran-markierte Endosome in Anwesenheit von Vasopressin
c SEM-Aufnahme im Fall a
d SEM-Aufnahme im Fall b
e FITC-Dextran-markierte Endosome in Anwesenheit von Forskolin
f FITC-Dextran-markierte Endosome in Anwesenheit von Choleratoxin
g FITC-Dextran-markierte Endosome in Anwesenheit von Vasopressin und V₂-Rezeptorantagonist H-9400.

### Experiment 1

Für die Untersuchung wurden Meerschweinchen mit einem normalen Preyer-Reflex, die ein Gewicht zwischen 300 und 500 g aufwiesen, benutzt. Zur Untersuchung der akuten Wirkung von Vasopressin wurde Pitressin® (Arginin-Vasopressin AVP) von Sankyo, Japan, intraperitoneal (0,2 Einheiten/g) injiziert. Die Meerschweinchen wurden für die Histologie zwei Stunden nach der Injektion getötet. Für die chronischen Experimente wurden 0,5 Einheiten/g des Vasopressins 60 Tage lang einmal pro Tag subkutan verabreicht. Für die Untersuchung der akuten Wirkung wurden 20 Tiere, für die Untersuchung der chronischen Wirkung 10 Tiere benutzt. In 10 Kontrolltiere wurden zum Vergleich 0,2 ml physiologische Kochsalzlösung intraperitoneal injiziert. Die Cochleae aller Versuchstiere wurden in Celloidin eingebettet und die mittig-modiolaren Schnitte wurden mit Haematoxylin/Eosin (HE) angefärbt. Aufgrund der Auslenkung der Reissner-Membran wurde das Vorliegen eines endolymphatischen Hydrops bestimmt.

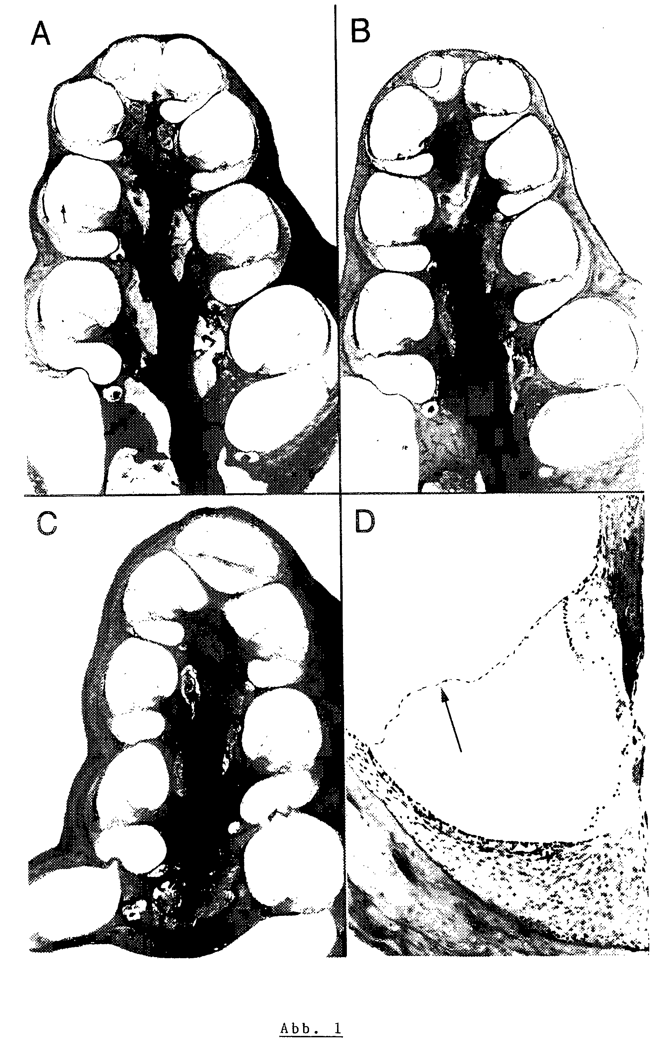

Die Ergebnisse von Experiment 1 sind in Abb. 1 dargestellt.

Abb. 1a zeigt, daß die beispielhaft mit einem Pfeil gekennzeichnete Reissner-Membran in den Kontrolltieren (n = 10) nicht ausgelenkt ist und dementsprechend auch kein Endolymphhydrops vorliegt.
Gemäß Abb. 1b ist bei einem Versuchstier mit chronischer Verabreichung von Vasopressin (n = 10) ein starker endolymphatischer Hydrops nachweisbar aufgrund der starken Verschiebung der Reissner-Membran. In der Schneckenwindung, die der mit dem Pfeil gekennzeichneten in Abb. 1a entspricht, berührt die Reissner-Membran sogar die knochige Trennwand zwischen den Windungen 3 und 4. Von den chronisch mit Vasopressin behandelten Versuchstieren zeigten vier von zehn schwere Hydrops gemäß Abb. 1b und weitere drei zeigten leichte bis mäßige Hydrops.
Abb. 1c zeigt einen leichten bis mäßigen Endolymphhydrops in einem Versuchstier nach einmaliger Injektion von Vasopressin, d.h. akuter Behandlung. Bei n = 20 zeigten acht dieser zwanzig Versuchstiere derartige leichte bis mäßige Hydrops. Abb. 1d zeigt den gleichen Fall wie Abb. 1c, jedoch bei höherer Vergrößerung. Im Gegensatz zu Abb. 1b ist kein Kontakt mit der knochigen Trennwand nachweisbar, jedoch deutliche Ausstülpungen der Reissner-Membran.

Experiment 1 und die zugehörige Abb. 1 zeigen somit, daß erhöhte Plasma-Werte von Vasopressin einen Endolymphhydrops hervorrufen können.

### Experiment 2

Mit den Primern AQP2s, AQP2as, V2s und V2as wurden PCR (Polymerase-Chain-Reaction)-Experimente durchgeführt. Dabei besaßen die Primer die folgenden Nukleotidsequenzen

AQP2s GAT CGC CGT GGC CTT TGG TCT

AQP2as AGG GAG CGG GCT GGA TTC AT

V2s AGT GCT GGG GGC CCT AAT ACG

V2as CAA ATC GGG CCC AGC AAT CAA ACA

Die cDNAs von Aquaporin-2 und dem V₂-Rezeptor wurden durch Verwendung der Primer-Paare AQP2s/AQP2as bzw. V2s/V2as amplifiziert. Die PCR wurden in einem Gesamtvolumen von 50 µl ausgeführt, die 5 µl der reversen Transkriptase, jeweils 0,8 µM der Primer, jeweils 200 µM der dNTPs, einen Inkubationspuffer (enthaltend 1,5 mM MgCl₂, von Pharmacia) und 1,25 U Taq-Polymerase (ebenfalls von Pharmacia) enthielt. Nach einem Denaturierungsschritt bei 94 °C von 7,5 min zu Beginn folgten 40 Zyklen von 50 sec bei 94 °C, 50 sec bei 55 °C und 50 sec bei 72 °C und einem zehnminütigen Schritt bei 72 °C zum Ende. Die erwarteten Längen der Produkte waren 428 bp bzw. 419 bp. Die PCR-Produkte wurden in üblicher Weise aufgearbeitet und durch Subcloning und Sequenzierung nachgewiesen.

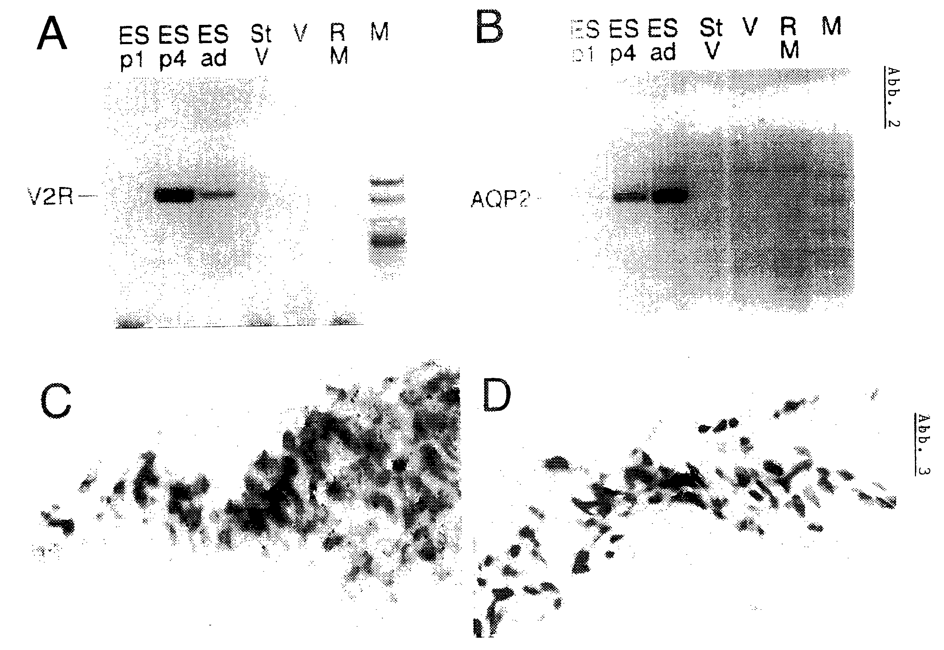

Wie aus Abb. 2 ersichtlich ist, werden sowohl V₂-Rezeptor als auch Aquaporin-2 im Epithel des endolymphatischen Sacks stark exprimiert, während in anderen Epithelien des Innenohrs, die ebenfalls mit der Endolymphe in Kontakt stehen, ein derartiger Nachweis nicht gelingt.

Gemäß Abb. 2a konnte im Innenohr der Ratte der V₂-Rezeptor sowohl am postnatalen Tag 4 (p4) und in der erwachsenen Ratte (ad) nachgewiesen werden. Sehr schwache Bande wurden im endolymphatischen Sack am postnatalen Tag 1 (p1), in der Stria vascularis (StV), im vestibulären Organ (V) oder in der Reissner-Membran (RM) erhalten. Gemäß Abb. 2b war die Expression von Aquaporin-2 am deutlichsten im erwachsenen Saccus endolymphaticus, klar detektierbar am postnatalen Tag 4, während jedoch keine Expression in der Stria vascularis, im vestibulären Organ oder in der Reissner-Membran nachgewiesen werden konnte.

### Experiment 3

Menschlicher Saccus endolymphaticus wurde von sechs Autopsien und zwei operierten Patienten mit Einwilligung der Verwandten bzw. der Patienten erhalten. Gefrorene Schnitte (20 µm) wurden auf einem Kryostaten bei -16 °C geschnitten, auf Gelatine-beschichtete Plättchen aufgebracht und über Nacht unter Vakuum bei 4 °C gelagert. Die Gewebeschnitte wurden mit ¹²⁵I-Arginin-Vasopressin über Nacht bei 4 °C inkubiert in Abwesenheit (totale Bindung) oder Anwesenheit von 10 µM von unmarkiertem Arginin-Vasopressin (unspezifische Bindung) und zwar in eiskaltem 10 mM Tris-HCl Puffer (pH 7,4), der 10 mg MgCl₂, 0,5 mg/ml Bacitracin und 0,1 % Rinderserumalbumin enthielt. Die radiomarkierten Schnitte wurden mit NTB-2 Nuklearemulsion (Eastman Kodak) beschichtet und für die lichtmikroskopische Autoradiographie präpariert. Die beschichteten Plättchen wurden in der Dunkelheit bei 4 °C für drei bis acht Tage gelagert. Nach Entwickeln und Fixieren wurden die Plättchen mit Haematoxylin/Eosin gefärbt.

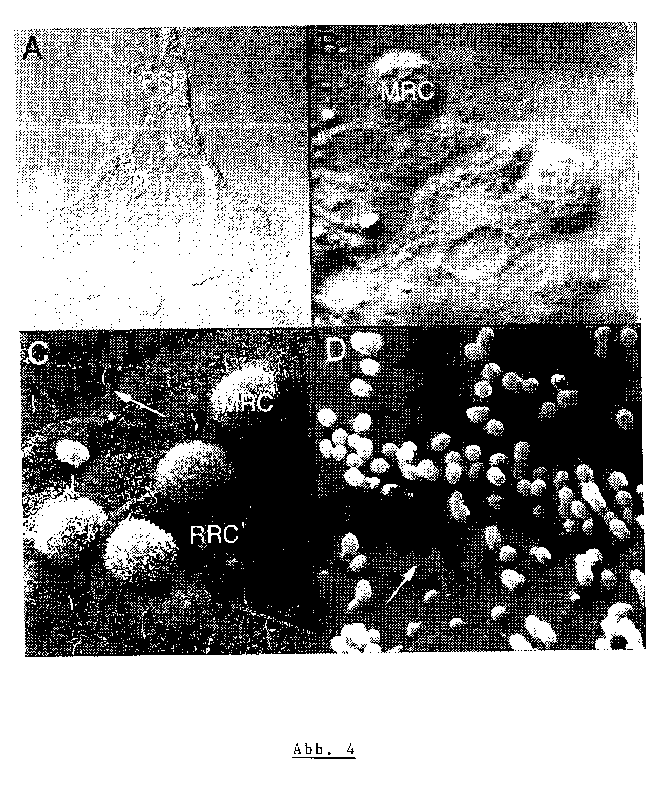

Abb. 3 zeigt die Ergebnisse von Experiment 3. Es ist die spezifische Bindung von radioaktivem Vasopressin im menschlichen endolymphatischen Sack zu erkennen. Die Punkte in Abb. 3c zeigen die Bindung des Vasopressins im Epithel des endolymphatischen Sacks, während gemäß Abb. 3d die gleiche Behandlung in Anwesenheit von nichtmarkiertem Vasopressin eine unspezifische Vasopressin-Bindung im Saccus ausschließt.

### Experiment 4

Ratten wurden am postnatalen Tag 4 durch Natrium-Pentobarbital (0,4 mg/gr Körpergewicht) betäubt und anschließend decapitiert. Die Schläfenbeine wurden sofort entfernt und in kalte (4 °C) HEPES-gepufferte Kochsalzlösung mit Hank's eingestellter Salzlösung (HHBSS) überführt. Der vollständige endolymphatische Sack wurde vom Schläfenbein getrennt, an der Ecke des distalen Saccus-Teils geöffnet und flach in ein Kulturplättchen eingesetzt, das mit 20 µl Cell Tek von Becton Dickinson Labware, USA, verdünnt 1:5, beschichtet war, und mit 300 µl Kulturmedium bedeckt. Das Kulturmedium bestand aus Minimum Essential Medium mit D-Valin, um das Wachstum von Fibroplasten zu unterdrücken und das mit 10 % Kalbsfötusserum, 10 mM HEPES, 100 IU/ml Penicillin und 2 mM-Glutamin ergänzt war. Die Kulturen wurden in einer 5 % Kohlendioxid-Atmosphäre bei 37 °C für bis zu 5 Tage gehalten. Die Morphologie der Kultur wurde durch Infrarotlicht-Mikroskopie beobachtet. Eine detaillierte Oberflächenmorphologie der Epithelien wurde durch SEM (Scanning-Electron-Microskopy) erhalten. Die Coverslips der Explantate wurden in 2,5 % Glutaraldehyd, 0,1 M Natriumkakodylat-Puffer 120 min lang fixiert, in 1 % Osmiumtetroxid 60 min nachfixiert, gewaschen, getrocknet, nach Standardvorgang goldbeschichtet und in einem Hitachi 500-SEM untersucht.

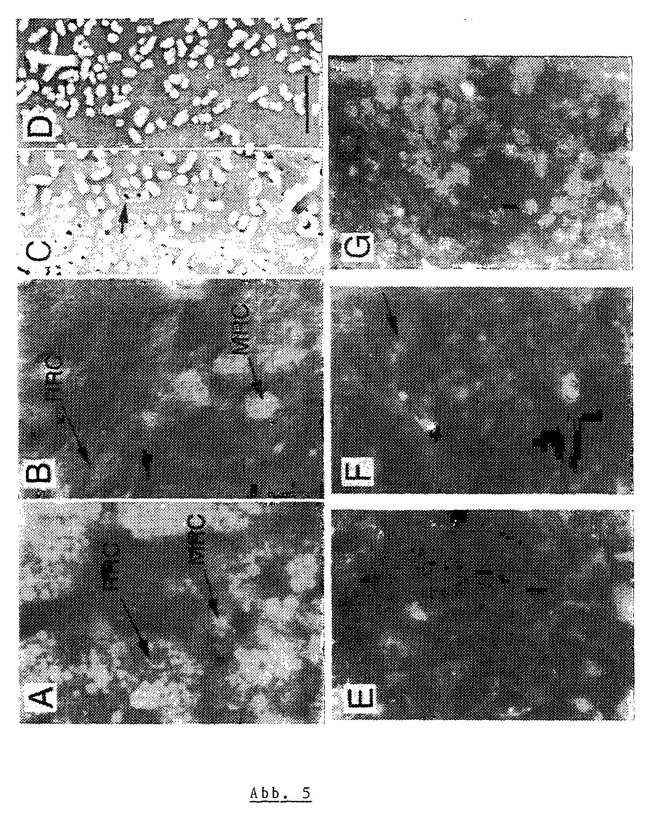

Abb. 4 zeigt die Ergebnisse von Experiment 4.

In Abb. 4a ist eine Übersicht auf einen Saccus endolymphaticus nach vier Tagen in Kultur dargestellt, wobei proximale (PSP), intermediäre (ISP) und distale (DSP) Saccus-Teile dargestellt sind. Die in Abb. 4b und 4c gezeigte Strukturanalyse des Kultur-Epithels des Saccus endolymphaticus zeigt eine deutliche Ähnlichkeit zum nativen Organ mit Mitochondrien- und Ribosomen-reichen Zellen typischer Gestalt. So zeigt die Aufnahme des Infrarotlicht-Mikroskops einzelne Zellen im intermediären Teil, wobei zwei Zelltypen nach Gestalt- und Oberflächenmorphologie unterschieden werden können. Die polygonal geformten Zellen, die den Ribosomen-reichen Zellen (RRC) entsprechen, haben eine flache Oberfläche, während die runden Zellen, die den Mitochondrien-reichen Zellen (MRC) entsprechen, zahlreiche in das Lumen hineinragende Mikrovilli besitzen. Dies ist anhand der SEM-Aufnahme gemäß Abb. 4c ebenfalls deutlich zu erkennen. Die höhere Vergrößerung gemäß Abb. 4d zeigt zusätzlich deutlich die Clathrin-beschichteten Löcher der luminalen Zellmembran in den RRC-Zellen des Saccus endolymphaticus (sh. Pfeil).

### Experiment 5

Bei einer Kultur gemäß Experiment 4 wurde nach zwölfstündiger Kultur der endolymphatische Sack in HHBSS (pH 7,3), das 1,0 mg/ml Fluorescein-Isothiocyanat (FITC)-Dextran (von Sigma, Deutschland) enthielt, bei 37 °C für etwa 10 min inkubiert. Anschließend wurde der endolymphatische Sack mit HHBSS gewaschen und mit 4 % Paraformaldehyd in PBS 20 min lang fixiert. Fluoreszenz- und Interferenzkontrast-Bilder wurden durch ein Epifluoreszenz-Mikroskop (Olympus, AX-70, Deutschland) mit einem Standard FITC-Filterset (Anregung: 485 ± 20 nm; Emission: > 510 nm) aufgenommen und übereinandergelegt, um auch die nichtfluoreszierenden Zellen sichtbar zu machen und die Mitochondrien-reichen und Ribosomen-reichen Zellen zu diskriminieren.
Anschließend wurden Vasopressin, Forskolin, Choleratoxin (alle von Sigma, Deutschland) oder der V₂-Rezeptorantagonist H-9400 (BACHEM, Schweiz) zu den Lösungen zusammen mit FITC-Dextran zugegeben, und zwar in den im folgenden noch beschriebenen Mengen.

In Abb. 5 sind die Ergebnisse von Experiment 5 dargestellt.

So zeigt Abb. 5a die durch die FITC-Dextran markierten Endosome repräsentierte Endozytose, die in der Kultur des endolymphatischen Sacks in den RRC und MRC bei Abwesenheit weiterer Substanzen, d.h. in einem Kontrollexperiment (n = 120) beobachtet wird. Bei Zugabe von 1 nM Vasopressin (n = 84) wird der Membranumsatz in RRC inhibiert, d.h. es sind keine markierten Endosome in RRC sichtbar. In MRC werden markierte Endosome immer noch beobachtet. Dies bedeutet, daß im endolymphatischen Sack das Vasopressin (im Gegensatz zu der Situation im Epithel des Sammelrohrs der Niere) die Aufnahme von FITC-Dextran in Ribosomen-reiche Zellen (RRC) inhibiert. So zeigen beim Beispiel gemäß Abb. 5b 10,5 ± 2,1 von 118,5 ± 2,8 Zellen FITC-Dextran-Aufnahme (n = 20), verglichen mit einer unbehandelten Probe nach Beispiel von Abb. 5a, bei dem 90,5 ± 2,5 von 116,5 ± 2,4 Zellen FITC-Dextran-Aufnahme (bei n = 20) zeigten.

Der inhibotorische Effekt des Vasopressins auf den Membranumsatz wird ebenfalls demonstriert durch das Verschwinden der Clathrin-beschichteten Löcher (pits) von der apikalen Zelloberfläche der Ribosomen-reichen Zellen gemäß den SEM-Aufnahmen von Abb. 5c und 5d. So zeigte RRC unter Kontrollbedingungen zahlreiche beschichtete Löcher (sh. Pfeil in Abb. 5c), die bereits in Abb. 4d bei höherer Vergrößerung dargestellt wurden. Der Querbalken in Abb. 5d repräsentiert eine Länge von 1 µm. Gemäß Abb. 5d sind nach einer Behandlung mit 1 mM Vasopressin fast keine Löcher mehr sichtbar, was die Internalisierung des vermutlich mit Aquaporin-2 geclusterten Clathrins nahelegt.

Gemäß Abb. 5e und 5f wurden, wie im Fall des Vasopressins, ebenfalls fast keine Endosome nachgewiesen bei Anwendung von 50 µM Forskolin (n = 48) bzw. 0,1 nM Choleratoxin (n = 36).

Genauso überraschend wie das Ergebnis des in Abbildung 5b dargestellten Experiments ist das Versuchsergebnis gemäß Abb. 5g, bei dem eine gleichzeitige Anwendung von 10 nM Vasopressin und 10 nM V₂-Rezeptorantagonist H-9400 den Vasopressin-Effekt gemäß Abb. 5b aufhebt. Die FITC-Dextran gefüllten Endosome sind weiterhin vorhanden (Versuchszahl n = 30).

Die geschilderten FITC-Dextran-Versuche machen sich die bekannte Tatsache zunutze, daß der Membranumsatz durch FITC-Dextran dargestellt werden kann und mit dem Wassertransport durch die Membran korreliert. Ein hoher durch FITC-Dextran nachgewiesener Membranumsatz läßt auf einen hohen Wassertransport schließen. Da das Epithel des endolymphatischen Sacks nahezu ausschließlich aus RRC- und MRC-Zellen besteht, ist der gemäß Experiment 5 geführte Nachweis aussagekräftig für den endolymphatischen Sack insgesamt und den zuführenden Ductus. Die Ergebnisse stehen auch in Übereinstimmung mit der Tatsache, daß Vasopressin an den RRC-Zellen aktiv ist und deshalb dort der Effekt von Vasopressin bzw. Vasopressin-Antagonist nachweisbar ist. Die MRC-Zellen sind nicht aktiv mit Vasopressin und zeigen in Übereinstimmung damit auch keinen Effekt gemäß Experiment 5.

Aufgrund der Tatsache, daß der verwendete peptidische Antagonist H-9400 ein vergleichsweise selektiver V₂-Rezeptorantagonist ist, stellen die Versuchsergebnisse einen starken Hinweis darauf dar, daß der Vasopressin-Rezeptor am endolymphatischen Sack des Innenohrs vom V₂-Typ ist. Erstaunlicherweise besitzt jedoch das Vasopressin im Innenohr offensichtlich eine umgekehrte Wirkung wie in den Epithelzellen des Sammelrohrs der Niere. Damit läßt sich auch das überraschende Ergebnis erklären, daß der Vasopressin-Rezeptorantagonist den Membranumsatz und damit den Wassertransport im Gegensatz zu den bekannten Wirkungen in der Niere erhöht und damit durch Verwendung des Antagonisten eine Wasser resorbierende Wirkung erzielt wird. Diese systematische Erkenntnis macht die erfindungsgemäße Verwendung der Vasopressin-Rezeptorantagonisten zur Behandlung von Erkrankungen oder Störungen des Innenohrs, insbesondere solcher, die mit einem Hydrops, wie einem Endolymphhydrops, verbunden sind, möglich. Eine mit einer Volumenabnahme auf der luminalen Seite verbundene Wirkung des Antagonisten führt im Innenohr, im Gegensatz zu der bekannten Wirkung in der Niere, bei vorhandenem Überdruck oder bei vorhandenem zu großem Volumen zu einer Druck- und Volumenabnahme. Diese sind geeignet die Symptome, also insbesondere Schwindel, Schwerhörigkeit und Tinnitus, zu lindern oder zu beseitigen. Der erfindungsgemäßen Verwendung kann auch eine prophylaktische Wirkung bei derartigen Innenohrstörungen zukommen.

## Patentansprüche

1. Verwendung mindestens eines Vasopressin-Rezeptorantagonisten oder Mischungen solcher Antagonisten zur Herstellung eines Medikamentes oder einer pharmazeutischen Zusammensetzung zur Behandlung von Störungen oder Erkrankungen des Innenohres.

2. Verwendung nach Anspruch 1, **dadurch gekennzeichnet, dass** es sich bei dem Rezeptorantagonisten um einen Vasopressin-V₂-Rezeptorantagonisten handelt.

3. Verwendung nach Anspruch 1 oder Anspruch 2, **dadurch gekennzeichnet, dass** die Störung oder Erkrankung des Innenohres mit mindestens einem der Symptome Schwindel, Schwerhörigkeit oder Tinnitus verbunden ist, wobei es sich bei der Schwerhörigkeit vorzugsweise um eine Tieftonschwerhörigkeit handelt.

4. Verwendung nach einem der vorhergehenden Ansprüche, **dadurch gekennzeichnet, dass** die Störung oder Erkrankung des Innenohres mit einem Hydrops, insbesondere einem Endolymphydrops in Zusammenhang steht.

5. Verwendung nach einem der vorhergehenden Ansprüche, **dadurch gekennzeichnet, dass** es sich bei der Störung oder Erkrankung des Innenohres um Morbus Ménière handelt.

6. Verwendung nach einem der vorhergehenden Ansprüche, **dadurch gekennzeichnet, dass** es sich bei dem Rezeptorantagonisten um eine Peptidverbindung handelt, wobei die Peptidverbindung vorzugsweise ein lineares Peptid, insbesondere Propionyl-D-Tyr(Et)-Phe-Val-Asn-Abu-Pro-Arg-Arg-NH₂, ist.

7. Verwendung nach einem der Ansprüche 1 bis 5, **dadurch gekennzeichnet, dass** es sich bei dem Rezeptorantagonisten um eine nicht-peptidische organische Substanz handelt.

8. Verwendung nach Anspruch 7, **dadurch gekennzeichnet, dass** die organische Substanz ein Benzazepin-Derivat ist, wobei das Benzazepin-Derivat vorzugsweise 5-Dimethylamino-1-{4-(2methylbenzoylamino)benzoyl}-2,3,4,5-tetrahydro-1H-benzazepin ist.

9. Verwendung nach Anspruch 7, **dadurch gekennzeichnet, dass** die organische Substanz ein Indol-Derivat ist, wobei das Indol-Derivat vorzugsweise 1-[4-(Ntert-butylcarbamoyl)-2-methoxylbenzolsulfonyl]-5-ethoxy-3-spiro-[4-(2-morpholinoethoxy)cyclohexan]indol-2-one, fumarat ist.

10. Verwendung nach einem der vorhergehenden Ansprüche, **dadurch gekennzeichnet, dass** der Rezeptorantagonist oral und/oder intravenös, insbesondere oral, verabreichbar ist.

11. Verwendung nach einem der vorhergehenden Ansprüche, **dadurch gekennzeichnet, dass** der Rezeptorantagonist in einer Menge von 0,1 bis 50 mg/kg Körpergewicht und pro Tag vorgesehen ist.

12. Verwendung nach einem der vorhergehenden Ansprüche, **dadurch gekennzeichnet, dass** der Rezeptorantagonist in einer zur Verabreichung vorgesehenen Zusammensetzung bzw. in einem zur Verabreichung vorgesehenen Medikament in einer Menge von 1 bis 75 Gew%, vorzugsweise 5 bis 50 Gew%, vorzugsweise 5 bis 25 Gew%, enthalten ist.

## Claims

1. Use of at least one vasopressin receptor antagonist or mixtures of such antagonists for producing a medicament or a pharmaceutical composition for the treatment of disorders or diseases of the inner ear.

2. Use according to Claim 1, **characterized in that** the receptor antagonist is a vasopressin V₂ receptor antagonist.

3. Use according to Claim 1 or Claim 2, **characterized in that** the disorder or disease of the inner ear is associated with at least one of the symptoms vertigo, hearing impairment or tinnitus, where the hearing impairment is preferably a low-frequency hearing impairment.

4. Use according to any of the preceding claims, **characterized in that** the disorder or disease of the inner ear is associated with a hydrops, especially an endolymphatic hydrops.

5. Use of according to any of the preceding claims, **characterized in that** the disorder or disease of the inner ear is Ménière's disease.

6. Use according to any of the preceding claims, **characterized in that** the receptor antagonist is a peptide compound where the peptide compound is preferably a linear peptide, in particular propionyl-D-Tyr(Et)-Phe-Val-Asn-Abu-Pro-Arg-Arg-NH₂.

7. Use according to any of Claims 1 to 5, **characterized in that** the receptor antagonist is a non-peptide organic substance.

8. Use according to Claim 7, **characterized in that** the organic substance is a benzazepine derivative, where the benzazepine derivative is preferably 5-dimethylamino-1-{4-(2-methylbenzoylamino)-benzoyl}-2,3,4,5-tetrahydro-1H-benzazepine.

9. Use according to Claim 7, **characterized in that** the organic substance is an indole derivative, where the indole derivative is preferably 1-[4-(N-tert-butylcarbamoyl)-2-methoxybenzenesulphonyl)-5-ethoxy-3-spiro-[4-(2-morpholinoethoxy)-cyclohexane]indol-2-one, fumarate.

10. Use according to any of the preceding claims, **characterized in that** the receptor antagonist can be administered orally and/or intravenously, especially orally.

11. Use according to any of the preceding claims, **characterized in that** the receptor antagonist is provided in an amount of from 0.1 to 50 mg/kg of body weight and per day.

12. Use according to any of the preceding claims, **characterized in that** the receptor antagonist is present in a composition intended for administration or in a medicament intended for administration in an amount of from 1 to 75% by weight, preferably 5 to 50% by weight, preferably 5 to 25% by weight.

## Revendications

1. Utilisation d'au moins un antagoniste de récepteur de vasopressine ou de mélanges de tels antagonistes pour la fabrication d'un médicament ou d'une composition pharmaceutique destinés au traitement de troubles ou de maladies de l'oreille interne.

2. Utilisation selon la revendication 1, **caractérisée en ce que** l'antagoniste de récepteur de vasopressine consiste en un antagoniste de récepteur de vasopressine V₂.

3. Utilisation selon la revendication 1 ou la revendication 2, **caractérisée en ce que** le trouble ou la maladie de l'oreille interne est associé à au moins l'un des symptômes suivants : vertiges, difficulté d'audition ou bourdonnements, la difficulté d'audition étant de préférence une difficulté d'audition dans les graves.

4. Utilisation selon l'une quelconque des revendications précédentes, **caractérisée en ce que** le trouble ou la maladie de l'oreille interne est en relation avec un hydrops, en particulier un hydrops endolymphatique.

5. Utilisation selon l'une quelconque des revendications précédentes, **caractérisée en ce que** le trouble ou la maladie de l'oreille interne est la maladie de Ménière.

6. Utilisation selon l'une quelconque des revendications précédentes, **caractérisée en ce que** l'antagoniste de récepteur consiste en un composé peptidique, le composé peptidique étant de préférence un peptide linéaire, en particulier le propionyl-D-Tyr(Et)-Phe-Val-Asn-Abu-Pro-Arg-Arg-NH₂.

7. Utilisation selon l'une quelconque des revendications 1 à 5, **caractérisée en ce que** l'antagoniste de récepteur est une substance organique non peptidique.

8. Utilisation selon la revendication 7, **caractérisée en ce que** la substance organique est un dérivé de benzazépine, le dérivé de benzazépine étant de préférence la 5-diméthylamino-1-{4-(2-méthylbenzoylamino)benzoyl)-2,3,9,5-tétrahydro-1H-benzazépine.

9. Utilisation selon la revendication 7, **caractérisée en ce que** la substance organique est un dérivé d'indole, le dérivé d'indole étant de préférence le fumarate de 1-[4-(Ntert-butylcarbamoyl)-2-méthoxybenzènesulfonyl]-5-éthoxy-3-spiro-[4-(2-morpholinoéthoxy)cyclohexane]indol-2-one.

10. Utilisation selon l'une quelconque des revendications précédentes, **caractérisée en ce que** l'antagoniste de récepteur peut être administré par voie orale et/ou intraveineuse, en particulier orale.

11. Utilisation selon l'une quelconque des revendications précédentes, **caractérisée en ce que** l'antagoniste de récepteur est prévu en une quantité de 0,1 à 50 mg/kg de poids corporel et par jour.

12. Utilisation selon l'une quelconque des revendications précédentes, **caractérisée en ce que** l'antagoniste de récepteur est contenu en une quantité de 1 à 75 % en poids, de préférence de 5 à 50 % en poids, de préférence de 5 à 25 % en poids dans une composition prévue pour l'administration ou dans un médicament prévu pour l'administration.
